# EUROPÄISCHE PATENTSCHRIFT

(11) **EP 1 624 072 B1**
(45) Veröffentlichungstag und Bekanntmachung des Hinweises auf die Patenterteilung: **02.04.2014**
(21) Anmeldenummer: 05023845.0
(22) Anmeldetag: 02.06.2000
(51) Int. Cl.: C12Q 1/37, G01N 33/573

(54) **Kit zur Bestimmung der Aktivität der Faktor VII-aktivierenden Protease aus Proteinlösungen**
Kit for the determination of the activity of the Factor VII-activating protease in protein solutions
Kit pour la détermination de l'activité de la protéase qui active le Factor VII dans des solutions protéiques

(30) Priorität: 10.06.1999 DE 19926531; 17.05.2000 DE 10023923
(43) Veröffentlichungstag der Anmeldung: 08.02.2006
(62) Teilanmeldung aus: 00111738.1
(73) Patentinhaber: CSL Behring GmbH, 35041 Marburg (DE)
(72) Erfinder: Römisch, Jürgen, Dr., 2440 Gramatneusiedl (AT); Feußner, Annette, 35043 Marburg (DE); Stöhr, Hans-Arnold, 35083 Wetter (DE)

(56) Entgegenhaltungen:
- EP-A- 0 952 215
- EP-A- 1 074 615
- EP-A- 1 074 616
- ROEMISCH J ET AL: "A PROTEASE ISOLATED FROM PLASMA WHICH ACTIVATES FVII IN A TISSUE FACTOR INDEPENDENT MANNER BU INACTIVATES FV AND FVIII" ANNALS OF HEMATOLOGY, BERLIN, DE, Bd. 78, Nr. SUPPL 1, 24. Februar 1999 (1999-02-24), Seite A10, XP001059496 ISSN: 0939-5555

## Beschreibung

Gegenstand der Erfindung ist ein Kit zur qualitativen und quantitativen Bestimmung der Faktor VII-aktivierenden Protease in komplexen Proteinlösungen wie Plasma.

Aus der deutschen Patentanmeldung 199 03 693.4 sind bereits Testsysteme und Verfahren zum qualitativen und quantitativen Nachweis einer Protease bekannt, die den Blutgerinnungsfaktor VII aktiviert. Diese umfassen chromogene Testverfahren, die auf der Spaltung markierter, niedermolekularer Peptidsubstrate und der photometrischen Bestimmung der dabei auftretenden Extinktion beruhen, sowie Testverfahren, bei denen die biologischen Eigenschaften der genannten Protease ausgenutzt werden. Dabei kann die Protease oder ihr Proenzym dadurch nachgewiesen werden, dass sie
a) eine die Blutgerinnungsfaktoren VIII/VIIIa oder V/Va inaktivierende Wirkung oder
b) in globalen Gerinnungstests eine die Blutgerinnungszeiten verkürzende Wirkung oder
c) eine Plasminogen-Aktivatoren aktivierende Wirkung oder
d) eine den FVII aktivierende Wirkung aufweist.

Die Bestimmung der Aktivität des FVII-Aktivators führt bei den bisher eingesetzten Bestimmungsverfahren jedoch nur dann zu zuverlässigen Ergebnissen, wenn die Protease in gereinigtem oder angereichertem Zustand vorliegt und keine störenden Einflüsse von Verunreinigungen das Messergebnis verfälschen. Besonders sehr komplexe Proteingemische wie Plasma oder Gewebeflüssigkeiten enthalten nun allerdings eine Vielzahl von Proteinen, die eine spezifische Bestimmung des Faktor VII-Aktivators verhindern oder zumindest behindern können. Außerdem liegt die Protease nach dem derzeitigen Wissensstand im Plasma vor allem als Proenzym vor, so dass eine Aktivierung zur aktiven Protease zum Zwecke der anschließenden Aktivitätsbestimmung nötig ist.

Die Faktor VII-aktivierende Protease ist ein Plasmaprotein, das an der Regulation der Hämostase, vor allem durch Mitwirkung an Gerinnungs- und Fibrinolyseprozessen, beteiligt ist. Deshalb ist die Untersuchung der Funktion und der biologischen Aktivität der Protease von hohem Interesse. So könnte zum Beispiel ein erniedrigter Antigengehalt und/oder eine Störung der biologischen Aktivität, beispielsweise durch eine Genmutation, ein erhöhtes Thromboserisiko anzeigen. Es stellte sich deshalb die Aufgabe, ein Verfahren zu entwickeln, das die möglichst einfache und spezifische Bestimmung einer oder mehrerer biologischer Aktivitäten der Faktor VII-aktivierenden Protease ermöglicht. Da die physiologische Aufgabe dieser Protease bisher noch unklar ist, sollte diese Methode prinzipiell die Bestimmung mehrerer Aktivitäten erlauben.

Es wurde nun gefunden, dass diese Anforderungen durch ein Verfahren zur Bestimmung der Aktivität der den Blutgerinnungsfaktor VII aktivierenden Protease in Proteinlösungen erfüllt werden, bei dem
- die der Protease und/oder ihr Proenzym enthaltende Proteinlösung mit einer Festphase inkubiert wird, an die zuvor ein gegen die Protease gerichteter Antikörper gekoppelt wurde und
- nach Auswaschen der Festphase die daran fixierte Protease und/oder ihr Proenyzm mit Reagenzien inkubiert werden, die deren Aktivitätsbestimmung erlauben.

Dieses Bestimmungsverfahren lässt sich überraschenderweise nicht nur auf die Protease in ihrer aktivierten Form anwenden. Obwohl die bisherigen Untersuchungsergebnisse darauf schließen lassen, dass die Protease im Plasma hauptsächlich als Proenzym zirkuliert und es daher zu erwarten war, dass dieses nach Bindung an die oben genannte Festphase erst noch aktiviert werden muss, um danach ihre biologischen Aktivitäten entfalten zu können, wurde nun überraschenderweise gefunden, dass eine solche Aktivierung nicht notwendig ist, sondern dass das aus dem Plasma an die Festphase gebundene Proenzym in immobilisierter Form seine biologische Aktivität in gleicher Weise entfaltet wie das aktive Enzym. Dadurch erübrigt sich ein gesonderter Aktivierungsschritt und ermöglicht eine weitaus schnellere und störungsfreiere Bestimmung.

Eine spezifische Aktivierung des Proenzyms kann aber durchaus noch angeschlossen werden, um sicherzustellen, dass das gebundene Proenzym vollständig aktiviert wurde.

Als Festphase kommen die dem Fachmann bekannten Matrices wie aktivierte Sepharose® oder Fraktogel® in Frage. Vorzugsweise werden Mikrotiterplatten mit gegen die Protease gerichteten Antikörpern beschichtet, die polyklonaler oder monoklonaler Herkunft sein können. Auch Antikörperfragmente wie F(ab) oder F(ab)₂ können verwendet werden. Im Gegensatz zum Antigentest, in dem ein markierter Zweitantikörper zur Detektion und dann auch zur Quantifizierung verwendet wird, werden erfindungsgemäß chromogene Substrate zugesetzt, die die Aktivitätsbestimmung der Protease zulassen. Ein besonders bevorzugtes chromogenes Substrat ist S2288 von Chromogenix AB (H-D-isoleucyl-L-prolyl-L-arginin-pNA x 2 HCl), das ebenso wie ähnliche Verbindungen eine signifikante konzentrations- und zeitabhängige Zunahme der Absorption durch Amidolyse des Substrats zeigt. Überraschenderweise behält die Protease ihre biologischen Aktivitäten und Eigenschaften auch nach Bindung an den Antikörper bei, nämlich die Fähigkeit FVII und Plasminogen-Aktivatören zu aktivieren. Dadurch wird die spezifische Bestimmung der Funktionalität der Protease aus einer komplexen Proteinlösung heraus möglich.

Neben den chromogenen Substraten bieten sich auch die übrigen in der deutschen Patentanmeldung 199 03 693.4 erwähnten Substrate zur Aktivitätsbestimmung an, also die Inaktivierung der Blutgerinnungsfaktoren VIII/VIIIa oder V/Va und auch die Aktivierung des FVII und der Plasminogenaktivatoren. Dabei kann zum Beispiel der so aktivierte Anteil an Faktor VII durch direkte Amidolyse eines für den FVII spezifischen chromogenen Substrats bestimmt werden oder durch eine gekoppelte Reaktion wie den sog. FVIIa-rTF-Test. Die Aktivierung von Einkettenplasminogenaktivatoren scuPA (single chain urokinase plasminogen activator) oder der sctPA (single chain tissue plasminogen activator) lässt sich einfach durch Substratumsetzung von zum Beispiel S2444 (pyroGlu-Gly-Arg-pNA x HCl) verfolgen. Wie in der deutschen Patentanmeldung 199 03 693.4 beschrieben, können zum Nachweis auch Substanzen zugesetzt werden, die die Aktivität der Protease stimulieren, zum Beispiel lösliche Kalziumsalze und/oder Heparin oder dem Heparin verwandte Substanzen wie Dextransulfat.

Weitere Untersuchungen von Lösungen, Körperflüsigkeiten und Zell- bzw. Gewebeextrakten mit Hilfe des beschriebenen Verfahrens zeigten dessen Eignung zur Detektion bzw. Quantifizierung der FVII-aktivierenden Protease. Darunter sind die diese Protease enthaltende Lösungen zu verstehen, wie Intermediate der Präparation der Protease, Zellkultur-Überstände, die auch bei der Fermentation entsprechender Zellen zur rekombinanten Expression anfallen. Darunter sind auch Lösungen zu verstehen, die bei der transgenen Herstellung der Protease bzw. des Proenzymes anfallen, wie Milch.

Darüber hinaus eignet sich das Verfahren zur Bestimmung der Aktivität der FVII-aktivierenden Protease in Extrakten von Geweben oder Zellen, die einen Eindruck über das Vorhandensein der Proteaseaktivität gibt bzw. über potentielle pathologische Zustände bei Über- oder Unterexpression dieses Proteins.

Ein besonderes Interesse gilt dem Nachweis der Proteaseaktivitäten in Körperflüssigkeiten, wie Blut und Plasma, Seminalplasma, Urin, Cerebrospinalflüssigkeit, Bronchioalveolar-Lavage, Fruchtwasser, Speichel oder Tränenflüssigkeit. Einen wertvolle Ergänzung zum Aktivitätstest stellt dabei das in der deutschen Patentanmeldung 199 03 693.4 erwähnte Antigenbestimmungssystem (z.B. ELISA) dar, da mit Hilfe beider Parameter ein umfassenderes Bild beispielsweise bei einer Erkrankung erhalten werden kann.

Bei der Untersuchung gesunder Blutspender fiel auf, daß ca. 5-10% der untersuchten Plasmen eine gegenüber einem Standard (Poolplasmen) deutlich geringere Proteaseaktivität aufwiesen (etwa 30-50% des 'Durchschnittswertes'), wie in Beispiel 1 näher ausgeführt. Diese Information wurde dadurch ergänzt, daß nahezu alle dieser Spender im Normalbereich befindliche Antigengehalte aufwiesen. Dies könnte beispielsweise auf (heterozygote) Mutation(en) hinweisen, die die Aktivität, jedoch nicht den Antigengehalt, einer Plasmaprobe entsprechend beeinflussen würden. Als Konsequenz könnten diese Donoren eine Risikogruppe für bestimmte Krankheiten darstellen und gegebenenfalls prophylaktische Maßnahmen frühzeitig ergriffen werden. Dies gilt sowohl für Personen, die erhöhte als auch erniedigte Aktivitätsspiegel aufweisen. Signifikant erhöhte Aktivitäten dieser Protease (bei teilweise normalem oder leicht erhöhten Antigengehalt) fanden wir in Plasmen von Patienten mit Herzinfarkten (siehe auch Beispiel 2) und stabiler und instabiler Angina pectoris verglichen mit einem Kollektiv gesunder Spender. Bislang ist noch nicht geklärt, ob eine Erhöhung der Proteaseaktivität als eine Ursache dieser Erkrankung gewertet werden kann oder ob diese eher einer Gegenreaktion des Körpers entspricht, im Sinne einer gesteigerten Thrombolyse. Abgesehen von der physiologischen Relevanz der erhöhten Proteaseaktivitäten kann dieser Parameter zur frühen Erkennung und als Kriterium einer Veränderung des Krankheitsbildes verwendet werden. Dies schließt die Diagnostik weiterer Herz-Kreislauf assoziierter Komplikationen ein.

Über diese Indikationen hinaus kann das beschriebene Testsystem (auch in Kombination mit einer Antigenbestimmung) zur Diagnose und Therapieverfolgung auch bei malignen Erkrankungen, Entzündungen, Autoimmunerkrankungen, Vaskulitiden, respiratorischen Defekten bzw. zur Hämostasediagnostik (Gerinnung und Fibrinolyse), wie auch bei Sepsis und assoziierten Reaktionen, wie der disseminierten intravasalen Gerinnung verwendet werden. Weitere Anwendungsgebiete umschließen die Diagnose von Organdefekten, wie zerebralen, respiratorischen und Nierenerkrankungen. In Patienten mit Leberzirrhose fanden wir signifikant erniedrigte Aktivitäten der Protease, die in den meisten Fällen mit erniedrigten Antigenspiegeln einhergingen.

Weitere Untersuchungen zeigten, daß neben einem moderaten Anstieg des Antigenspiegels im Plasma gesunder Schwangerer ein deutlicher Anstieg der Proteaseaktivit im Verlauf von Schwangerschaften zu beobachten ist, mit höchsten Werten im dritten Trimester. Ein Ausbleiben eines solchen Anstieges der Protease kann mit einem Risiko für Mutter und Kind während der Schwangerschaft verbunden sein, wie thromboembolischen Komplikationen etc. bis hin zur Früh- und Fehlgeburt oder Mißbildung des Fötus.

Die Erfindung wird durch die folgenden Beispiele erläutert:

### Beispiel 1

Mikrotiterplatten (96 wells) wurden mit einem monoklonalen Antikörper gegen den FVII-Aktivator beschichtet, indem in jede Vertiefung 150 µl einer 10 µg/ml enthaltenden Lösung pipettiert wurde. Nach 16-stündiger Inkubation bei Raumtemperatur wurde die Platte mehrmals gewaschen. In die Vertiefungen wurden jeweils 100 µl steigender Konzentrationen gereinigter Protease bzw. verschiedene Verdünnungen eines Standardhumanplasmas (SHPL) pipettiert. Nach Inkubation bei 37°C wurden die Lösungen durch mehrmaliges Waschen entfernt und die Aktivitäten bestimmt.

In jede Vertiefung wurden 50 µl einer Prourokinase-Lösung (10µg/ml, American Diagnostica, US) pipettiert sowie 50 µl Puffer, der 30 mM CaCl₂ und 100 IU/ml Heparin enthielt. Zwei Minuten später wurden weitere 100 µl Puffer und 25 µl des Substrates S2444 (3 mM) hinzugegeben. Die Zunahme der Absorption bei 405 nm pro Minute wurde ermittelt.

| **Protease, gereinigt (µg/ml)** | **Δ mOD/min** | **SHPL (Verdünnung)** | **Δ mOD/min** |
|---|---|---|---|
| 0 | 0,4 | buffer | 0,4 |
| 0,1 | 7 | 1:200 | 0,4 |
| 0,2 | 12 | 1:100 | 0,9 |
| 0,4 | 18 | 1:50 | 7,5 |
| 0,6 | 22 | 1:33,3 | 8,4 |
| 0,8 | 24 | 1:25 | 15,2 |
| 1,0 | 27 | 1:20 | 24,8 |
| 2,0 | 34 | 1:10 | 31,2 |

### Beispiel 2

Die Beschichtung der Mikrotiterplatten und die Inkubation mit den Probelösungen wurde durchgeführt wie in Beispiel 1 beschrieben. Anstelle der Aktivierung der Prourokinase wurde die Aktivierung des Faktors VII bestimmt. Dazu wurden je 50µl Puffer, enthaltend 30 mM CaCl₂ und 100 IU/ml Heparin, für 2 Minuten bei Raumtemperatur in die Vertiefungen der Platte gegeben. Nach Zusatz von weiteren 100 µl Puffer und 25µl Spectrozym® VIIa (3 mM, American Diagnostica/US) wurde die Δ mOD/min bestimmt.

| **Protease, gereinigt (µg/ml)** | **Δ mOD/min** | **SHPL (Verdünnung)** | **Δ mOD/min** |
|---|---|---|---|
| 0 | 0,3 | buffer | 0,3 |
| 0,2 | 1,8 | 1:100 | 0,3 |
| 0,4 | 2,8 | 1:50 | 0,3 |
| 0,6 | 3,0 | 1:33,3 | 0,8 |
| 0,8 | 3,6 | 1:25 | 3,2 |
| 1,0 | 4,7 | 1:20 | 7,2 |
| 1,5 | 7,1 | 1:13,3 | 8,4 |
| 2,0 | 7,9 | 1:10 | 11,5 |

Mit Hilfe dieser Verdünnungsreihen ist es möglich, individuelle Plasmen zu vergleichen und die Funktionalität der Protease zu bestimmen. Durch Vergleich mit einem Standardhumanplasma, das einen Pool von hunderten von Einzelplasmen darstellt, können signifikante Abweichungen von der Norm erfasst werden. Die so ermittelte Aktivität sollte idealerweise in das Verhältnis zum Antigengehalt gesetzt werden, der zum Beispiel mittels ELISA bestimmt werden kann.

Ist die Proteasemenge bekannt, dann lässt sich die spezifische Aktivität der in der Proteinlösung enthaltenen Protease sowie dessen Proenzyms bestimmen.

### Beispiel 3

### Bestimmung der Proteaseaktivität in 190 Plasmen gesunder Spender

190 Citrat-Plasmen gesunder Personen, davon 140 Männer und 50 Frauen, wurden mit Hilfe des beschriebenen Aktivitätstestes untersucht. Um festzustellen, ob potentiell vom Durchschnittswert aller untersuchten Plasmen abweichende Aktivitäten mit einer entsprechenden Veränderung des Protease Antigenspiegels einhergingen, wurde ein ELISA verwendet, wie in der deutschen Patentanmeldung 199 03 693.4 beschrieben. Ein solcher ELISA zum Nachweis der Protease als Antigen ist mit Hilfe monoklonaler oder polyklonaler spezifischer Antikörper gegen diese Protease machbar.

**Abbildung (1****)** zeigt die Proteaseaktivitäten der untersuchten gesunden Männer (A) und Frauen (B). Es wird deutlich, daß 5-10%, sowohl Männer als auch Frauen, eine gegenüber dem Durchschnitt deutlich verminderte Aktivität aufweisen.

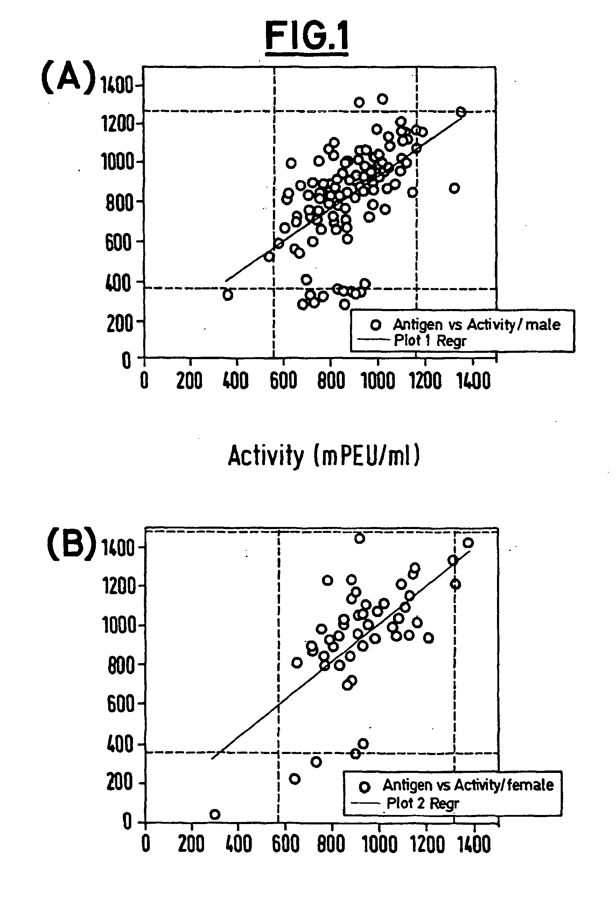

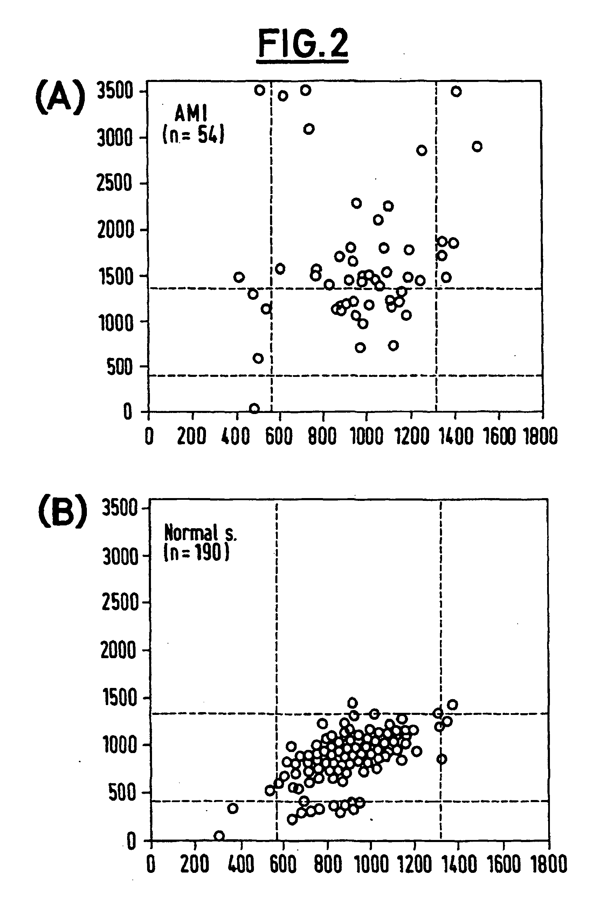

Die Proteaseaktivitäten (y-Achse) und die dazu gehörenden Antigenspiegel der entsprechenden Personen (x-Achse) sind in der Abbildung dargestellt. Die arbiträren 'Normalbereiche' der Antigen- und Aktivitätsspiegel sind durch waagerechte und senkrechte Linien als obere und untere Begrenzungen der Parameter jeweils gezeigt. Die sich daraus ergebenden Rechtecke (in jeweils der Mitte der Abbildung) stellen demnach die 'Normalbereiche' gesunder Spender dar. Hier wird nochmals besonders deutlich, daß die Mehrzahl der Proben mit verminderter Aktivität nicht mit einer entsprechenden Verminderung der Antigenspiegel einherging. Dies könnte auf eine heterozygote Mutation (oder mehrere) hinweisen, d.h. beispielsweise könnten ca. 50% der Proteasemoleküle durch eine oder mehrere Mutationen so verändert sein, daß eine Reaktion mit biologischen Substraten nicht mehr gewährleistet ist. Im Falle einer fibrinolytischen Bedeutung der Protease könnte dies mit einem Thromboserisiko (oder für andere Erkrankungen etc.) dieser derzeit noch 'gesunden' Population verbunden sein. Obwohl in der Minderzahl der untersuchten Proben, sind die Werte der reduzierten Proteaseaktivität, die sehr wohl mit einem verminderten Antigengehalt einhergehen, als nicht minder interessant zu bewerten, da offensichtlich eine Dysregulation der plasmatischen Verfügbarkeit der Protease vorliegt, die mit einem vergleichbaren Risiko wie beschrieben verbunden sein kann.

Entsprechend ist die Detektion der Proteaseaktivität, auch im Verbund mit der Antigenbestimmung, als ein Parameter zur Früherkennung und Prophylaxe-/Therapiekontrolle zu sehen.

### Beispiel 4

### Bestimmung der Proteaseaktivität in Plasmen schwangerer Frauen

Zitratplasmen schwangerer Frauen wurden, wie in Beispiel 3 beschrieben, getestet. Proben wurden an verschiedenen Zeitpunkten der Schwangerschaft gewonnen und anschließend untersucht.

Die Verläufe zweier unproblematischer Schwangerschaften sind in Tabelle 1 dargestellt. Ein deutlicher Anstieg der Proteaseaktivität mit Dauer der Schwangerschaft ist zu erkennen, wogegen die Antigegehalte der Protease nicht bis moderat ansteigen. Ein Ausbleiben dieser erhöhten Aktivität könnte mit Problemen für Mutter und Fötus verbunden sein.

Gesunde (nicht Schwangere) zeigen dagegen eine kontinuierlichen Verlauf der Proteaseaktivitäten (weder erhöht noch vermindert, im Rahmen der Testschwankungen) während einer entsprechend Beobachtungszeit (nicht dargestellt).

**Tabelle 1**

| Die prozentualen Angaben beziehen sich auf Durchschnittswerte gesunder (nicht schwangerer) Frauen. | | | | |
|---|---|---|---|---|
| | Antigen (%) | | Aktivität (%) | |
| Trimester der Schwangerschaft | Schwangere | | Schwangere | |
| | 1 | 2 | 1 | 2 |
| I | 103 | 105 | 110 | 115 |
| II | 118 | 123 | 158 | 176 |
| III | 126 | 143 | 215 | 280 |

### Beispiel 5

### Bestimmung der Proteaseaktivität in Herzinfarktplasmen

Plasmen von 54 Patienten mit akutem Myokardinfarkt wurden bei Einlieferung (vor Intensivbehandlung) in die Notfallstation gewonnen und für die Routineanalytik verwendet. Später wurden Plasmareste (nicht aufgetaute Aliquots) zur Quantifizirung der Proteaseaktivitäten (und Antigengehalte) verwendet.

Die **Abbildung (2****)** faßt die Ergebnisse der Untersuchung zusammen. Gegenüber einem Kollektiv gesunder Spender (**B**) sind in Plasmen von Patienten mit akutem Myokardinfarkt (**A**) signifikant höhere Proteaseaktivitäten (und auch der Antigengehalte) zu messen.

Entsprechend können diese Parameter zur Früherkennung eines Infarktes, also auch bei stabiler und instabiler Angina pectoris, verwendet werden. Bei Patienten mit diesen koronaren Herzerkrankungen fanden wir ebenfalls im Durchschnitt signififkant erhöhte Aktivitäten. Die Höhe der gemessenen Werte kann eine Bewertung des Schweregrades der Krankheit ermöglichen bzw. im Verlaufe einer Infarkt- und Angina pectoris Prophylaxe und Therapie wertvolle Hinweise über den Zustand des Patienten geben. Darüber hinaus können diese Parameter zur Beurteilung anderer mit dem Herz-Kreislaufsystem asoziierter Komplikationen verwendet werden.

## Patentansprüche

1. Kit zur Bestimmung der Aktivität der den Blutgerinnungsfaktor VII aktivierenden Protease **dadurch gekennzeichnet, dass** eine Festphase, an die zuvor ein gegen die Protease gerichteter Antikörper gekoppelt wurde, und ein chromogenes Substrat, Faktor VII, Faktor VIII/VIIIa, Faktor V/Va oder Plasminogenaktivatoren enthalten sind, wobei das chromogene Substrat eine Aktivitätsbestimmung der Protease durch eine konzentrations- und zeitabhängige Zunahme der Absorption durch Amidolyse des Substrats zuläßt.

2. Kit zur Bestimmung der Aktivität der den Blutgerinnungsfaktor VII aktivierenden Protease gemäß Anspruch 1 **dadurch gekennzeichnet, dass** der gegen die Protease gerichtete Antikörper ein polyklonaler oder monoklonaler Antikörper oder ein F(ab) oder F(ab)2-Antikörperfragment ist.

3. Kit zur Bestimmung der Aktivität der den Blutgerinnungsfaktor VII aktivierenden Protease gemäß den Ansprüchen 1 bis 2 **dadurch gekennzeichnet, dass** zusätzlich eine Substanz enthalten ist, die die Aktivität der Protease stimuliert.

4. Kit zur Bestimmung der Aktivität der den Blutgerinnungsfaktor VII aktivierenden Protease gemäß Anspruch 3 **dadurch gekennzeichnet, dass** die die Protease stimulierenden Substanzen lösliche Kalziumsalze und/oder Heparin oder dem Heparin verwandte Substanzen wie Dextransulfat sind.

5. Verfahren zur Früherkennung eines Infarktes oder von stabiler oder instabiler Angina Pectoris, **dadurch gekennzeichnet, dass** in einer Körperflüssigkeitsprobe eine signifikant erhöhte Aktivität der den Blutgerinnungsfaktor VII aktivierenden Protease gegenüber einem Kollektiv gesunder Spender bestimmt wird, wobei die Bestimmung der Aktivität der den Blutgerinnungsfaktor VII aktivierenden Protease dadurch erfolgt, dass
- die die Protease und/oder ihr Proenzym enthaltende Körperflüssigkeitsprobe mit einer Festphase inkubiert wird, an die zuvor ein gegen die Protease gerichteter Antikörper gekoppelt wurde und
- nach Auswaschen der Festphase die daran fixierte Protease und/oder ihr Proenzym mit Reagenzien inkubiert werden, die deren Aktivitätsbestimmung erlauben.

6. Verfahren zur Früherkennung eines Infarktes oder von stabiler oder instabiler Angina Pectoris gemäß Anspruch 5 weiterhin **dadurch gekennzeichnet, dass** ein signifikant erhöhter Antigengehalt der den Blutgerinnungsfaktor VII aktivierenden Protease bestimmt wird.

7. Verfahren zur Diagnose des Verlaufs und/oder des Schweregrades eines Infarktes und/oder des Erfolgs von Prophylaxe und/oder Therapie bei Angina Pectoris, **dadurch gekennzeichnet, dass** in einer Körperflüssigkeitsprobe die Aktivität der den Blutgerinnungsfaktor VII aktivierenden Protease bestimmt wird und die Höhe der gemessenen Werte Hinweise über den Zustand des Patienten gibt, wobei die Bestimmung der Aktivität der den Blutgerinnungsfaktor VII aktivierenden Protease dadurch erfolgt, dass
- die die Protease und/oder ihr Proenzym enthaltende Körperflüssigkeitsprobe mit einer Festphase inkubiert wird, an die zuvor ein gegen die Protease gerichteter Antikörper gekoppelt wurde und
- nach Auswaschen der Festphase die daran fixierte Protease und/oder ihr Proenzym mit Reagenzien inkubiert werden, die deren Aktivitätsbestimmung erlauben.

8. Verfahren zur Schwangerschaftsüberwachung, **dadurch gekennzeichnet, dass** mit Dauer der Schwangerschaft ein Anstieg der Aktivität der den Blutgerinnungsfaktor VII aktivierenden Protease bestimmt wird, wobei die Bestimmung der Aktivität der den Blutgerinnungsfaktor VII aktivierenden Protease zu verschiedenen Zeitpunkten der Schwangerschaft dadurch erfolgt, dass
- die die Protease und/oder ihr Proenzym enthaltene Körperflüssigkeitsprobe mit einer Festphase inkubiert wird, an die zuvor ein gegen die Protease gerichteter Antikörper gekoppelt wurde und
- nach Auswaschen der Festphase die daran fixierte Protease und/oder ihr Proenzym mit Reagenzien inkubiert werden, die deren Aktivitätsbestimmung erlauben.

9. Verfahren zur Schwangerschaftsüberwachung gemäß Anspruch 8 weiterhin
**dadurch gekennzeichnet, dass** ein moderater Anstieg des Antigengehalts der den Blutgerinnungsfaktor VII aktivierenden Protease bestimmt wird.

10. Verfahren zur Früherkennung eines Thromboserisikos, **dadurch gekennzeichnet, dass** in einer Körperflüssigkeitsprobe eine gegenüber dem Durchschnitt verminderte Aktivität der den Blutgerinnungsfaktor VII aktivierenden Protease bestimmt wird, wobei die Bestimmung der Aktivität der den Blutgerinnungsfaktor VII aktivierenden Protease dadurch erfolgt, dass
- die die Protease und/oder ihr Proenzym enthaltende Körperflüssigkeitsprobe mit einer Festphase inkubiert wird, an die zuvor ein gegen die Protease gerichteter Antikörper gekoppelt wurde und
- nach Auswaschen der Festphase die daran fixierte Protease und/oder ihr Proenzym mit Reagenzien inkubiert werden, die deren Aktivitätsbestimmung erlauben.

11. Verfahren zur Früherkennung eines Thromboserisikos gemäß Anspruch 10 weiterhin **dadurch gekennzeichnet, dass** ein verminderter oder normaler Antlgengehalt der den Blutgerinnungsfaktor VII aktivierenden Protease bestimmt wird.

## Claims

1. Kit for determining the activity of the protease activating blood coagulation factor VII, **characterized in that** it contains a solid phase to which an antibody directed against the protease has been pre-coupled and a chromogenic substrate, factor VII, factor VIII/VIIIa, factor V/Va or plasminogen activators, the chromogenic substrate allowing determination of the activity of the protease through a concentration- and time-dependent increase in absorption owing to amidolysis of the substrate.

2. Kit for determining the activity of the protease activating blood coagulation factor VII according to Claim 1, **characterized in that** the antibody directed against the protease is a polyclonal or monoclonal antibody or a F(ab) or F(ab)2 antibody fragment.

3. Kit for determining the activity of the protease activating blood coagulation factor VII according to Claims 1 to 2, **characterized in that** it additionally contains a substance which stimulates the activity of the protease.

4. Kit for determining the activity of the protease activating blood coagulation factor VII according to Claim 3, **characterized in that** the substances stimulating the protease are soluble potassium salts and/or heparin or heparin-related substances such as dextran sulphate.

5. Method for early detection of an infarction or of stable or unstable angina pectoris, **characterized in that** significantly increased activity of the protease activating blood coagulation factor VII with respect to a group of healthy donors is determined in a body fluid sample, the activity of the protease activating blood coagulation factor VII being determined by
- incubating the body fluid sample containing the protease and/or its proenzyme with a solid phase to which an antibody directed against the protease has been pre-coupled and
- washing out the solid phase and then incubating the protease and/or its proenzyme fixed thereto with reagents which allow determination of the activity thereof.

6. Method for early detection of an infarction or of stable or unstable angina pectoris according to Claim 5, additionally **characterized in that** a significantly increased antigen content of the protease activating blood coagulation factor VII is determined.

7. Method for diagnosing the progression and/or the severity of an infarction and/or the success of prophylaxis and/or therapy in the case of angina pectoris, **characterized in that** the activity of the protease activating blood coagulation factor VII is determined in a body fluid sample and the level of the measured values indicates the state of the patient, the activity of the protease activating blood coagulation factor VII being determined by
- incubating the body fluid sample containing the protease and/or its proenzyme with a solid phase to which an antibody directed against the protease has been pre-coupled and
- washing out the solid phase and then incubating the protease and/or its proenzyme fixed thereto with reagents which allow determination of the activity thereof.

8. Method for monitoring pregnancy, **characterized in that** a rise in the activity of the protease activating blood coagulation factor VII is determined over the course of the pregnancy, the activity of the protease activating blood coagulation factor VII being determined at different times during the pregnancy by
- incubating the body fluid sample containing the protease and/or its proenzyme with a solid phase to which an antibody directed against the protease has been pre-coupled and
- washing out the solid phase and then incubating the protease and/or its proenzyme fixed thereto with reagents which allow determination of the activity thereof.

9. Method for monitoring pregnancy according to Claim 8, additionally **characterized in that** a moderate rise in the antigen content of the protease activating blood coagulation factor VII is determined.

10. Method for early detection of the risk of thrombosis, **characterized in that** below-average activity of the protease activating blood coagulation factor VII is determined in a body fluid sample, the activity of the protease activating blood coagulation factor VII being determined by
- incubating the body fluid sample containing the protease and/or its proenzyme with a solid phase to which an antibody directed against the protease has been pre-coupled and
- washing out the solid phase and then incubating the protease and/or its proenzyme fixed thereto with reagents which allow determination of the activity thereof.

11. Method for early detection of the risk of thrombosis according to Claim 10, additionally **characterized in that** a reduced or normal antigen content of the protease activating blood coagulation factor VII is determined.

## Revendications

1. Nécessaire pour la détermination de l'activité de la protéase activant le facteur de coagulation VII, **caractérisé en ce que** sont contenus une phase solide, à laquelle a été couplé au préalable un anticorps dirigé contre la protéase, et un substrat chromogène, du facteur VII, du facteur VIII/VIIIa, du facteur V/Va ou des activateurs du plasminogène, le substrat chromogène permettant une détermination de l'activité de la protéase par une augmentation, dépendant du temps et de la concentration, de l'absorption par amidolyse du substrat.

2. Nécessaire pour la détermination de l'activité de la protéase activant le facteur de coagulation VII selon la revendication 1, **caractérisé en ce que** l'anticorps dirigé contre la protéase est un anticorps monoclonal ou polyclonal ou un fragment d'anticorps F(ab) ou F(ab)2.

3. Nécessaire pour la détermination de l'activité de la protéase activant le facteur de coagulation VII selon les revendications 1 et 2, **caractérisé en ce qu'**est également contenue une substance qui stimule l'activité de la protéase.

4. Nécessaire pour la détermination de l'activité de la protéase activant le facteur de coagulation VII selon la revendication 3, **caractérisé en ce que** les substances stimulant la protéase sont des sels de potassium solubles et/ou l'héparine ou des substances apparentées à l'héparine, telles que le sulfate de dextrane.

5. Procédé pour la détection précoce d'un infarctus ou d'un angor stable ou instable, **caractérisé en ce qu'**on détermine dans un échantillon de liquide corporel une activité significativement accrue de la protéase activant le facteur de coagulation VII, par rapport à un groupe de donneurs sains, la détermination de l'activité de la protéase activant le facteur de coagulation VII s'effectuant par
- mise en incubation de l'échantillon de liquide corporel contenant la protéase et/ou sa proenzyme avec une phase solide à laquelle a été couplé au préalable un anticorps dirigé contre la protéase et
- après extraction par lavage de la phase solide, mise en incubation de la protéase et/ou de sa proenzyme, fixée(s) sur celle-ci, avec des réactifs qui permettent la détermination de leur activité.

6. Procédé pour la détection précoce d'un infarctus ou d'un angor stable ou instable selon la revendication 5, en outre **caractérisé en ce qu'**on détermine une teneur significativement accrue en antigène de la protéase activant le facteur de coagulation VII.

7. Procédé pour le diagnostic de l'évolution et/ou du degré de gravité d'un infarctus et/ou du succès d'une prophylaxie et/ou d'une thérapie dans le cas d'angine de poitrine, **caractérisé en ce qu'**on détermine dans un échantillon de liquide corporel l'activité de la protéase activant le facteur de coagulation VII et la grandeur des valeurs trouvées donne une indication de l'état du patient, la détermination de l'activité de la protéase activant le facteur de coagulation VII s'effectuant par
- mise en incubation de l'échantillon de liquide corporel contenant la protéase et/ou sa proenzyme avec une phase solide à laquelle a été couplé au préalable un anticorps dirigé contre la protéase et
- après extraction par lavage de la phase solide, mise en incubation de la protéase et/ou de sa proenzyme, fixée(s) sur celle-ci, avec des réactifs qui permettent la détermination de leur activité.

8. Procédé pour la surveillance d'une grossesse, **caractérisé en ce qu'**on détermine avec la durée de la grossesse une augmentation de l'activité de la protéase activant le facteur de coagulation VII, en effectuant à divers moments de la grossesse la détermination de l'activité de la protéase activant le facteur de coagulation VII, par
- mise en incubation de l'échantillon de liquide corporel contenant la protéase et/ou sa proenzyme avec une phase solide à laquelle a été couplé au préalable un anticorps dirigé contre la protéase et
- après extraction par lavage de la phase solide, mise en incubation de la protéase et/ou de sa proenzyme, fixée(s) sur celle-ci, avec des réactifs qui permettent la détermination de leur activité.

9. Procédé pour la surveillance d'une grossesse selon la revendication 8, en outre caractérisé en qu'on détermine une augmentation modérée de la teneur en antigène de la protéase activant le facteur de coagulation VII.

10. Procédé pour la détection précoce d'un risque de thrombose, **caractérisé en ce qu'**on détermine dans un échantillon de liquide corporel une activité réduite par rapport à la moyenne de la protéase activant le facteur de coagulation VII, la détermination de l'activité de la protéase activant le facteur de coagulation VII s'effectuant par
- mise en incubation de l'échantillon de liquide corporel contenant la protéase et/ou sa proenzyme avec une phase solide à laquelle a été couplé au préalable un anticorps dirigé contre la protéase et
- après extraction par lavage de la phase solide, mise en incubation de la protéase et/ou de sa proenzyme, fixée(s) sur celle-ci, avec des réactifs qui permettent la détermination de leur activité.

11. Procédé pour la détection précoce d'un risque de thrombose selon la revendication 10, en outre **caractérisé en ce qu'**on détermine une teneur normale ou réduite en antigène de la protéase activant le facteur de coagulation VII.
